# EUROPÄISCHE PATENTSCHRIFT

(11) **EP 2 503 946 B1**
(45) Veröffentlichungstag und Bekanntmachung des Hinweises auf die Patenterteilung: **02.10.2013**
(21) Anmeldenummer: 11730569.8
(22) Anmeldetag: 27.06.2011
(51) Int. Cl.: A61B 17/54

(54) **WERKZEUGAUFNAHME**
TOOL RECEPTACLE
LOGEMENT D'OUTIL

(30) Priorität: 01.07.2010 DE 102010025826
(43) Veröffentlichungstag der Anmeldung: 03.10.2012
(73) Patentinhaber: Ladwig Feinwerktechnik GmbH, 75181 Pforzheim (DE)
(72) Erfinder: LADWIG, Manfred, 75181 Pforzheim (DE)
(74) Vertreter: Wacker, Jost Oliver
(86) Internationale Anmeldenummer: PCT/EP2011/003147
(87) Internationale Veröffentlichungsnummer: WO 2012/000635

(56) Entgegenhaltungen:
- DE-A1- 10 130 898
- DE-U1- 29 716 551
- DE-U1-202006 006 551

## Beschreibung

Die Erfindung betrifft eine Werkzeugaufnahme eines medizinischen oder kosmetischen Bearbeitungsgerätes, wie insbesondere eines Schleifgerätes für Pediküre oder Podologie, nach dem Oberbegriff des Anspruchs 1. Diese weist eine Schnellspannvorrichtung für die Festlegung eines Werkzeugschaftes eines Rotationswerkzeuges mit einer Schaftaufnahme auf. Der freie Querschnitt dieser Schaftaufnahme ist dabei durch Klemmelemente verringerbar, um einen in die Schaftaufnahme eingeführten Werkzeugschaft festklemmen zu können. Die Klemmelemente sind hierzu in Ausnehmungen eines Klemmelementträgers gehalten, wobei der Klemmelementträger innerhalb einer Spannhülse angeordnet ist. Diese Spannhülse kann beispielsweise mittels eines Motors in Rotationsbewegung versetzt werden und weist auf Höhe der Klemmelemente eine Innenwandung auf, an der Einbuchtungen eingelassen sind, in die die Klemmelemente wenigstens teilweise eingerückt werden können. Die Innenwandung kann hierzu beispielsweise einen polygonalen Querschnitt aufweisen. Im eingerückten Zustand geben die Klemmelemente dabei die Schaftaufnahme soweit frei, dass ein Werkzeugschaft eingeführt werden kann beziehungsweise ein bereits aufgenommener Werkzeugschaft aus der Schaftaufnahme entfernt werden kann. Bei motorischem Antrieb der Spannhülse um eine Rotationsachse herum werden die Klemmelemente dagegen mittels der Innenwand aus den Einbuchtungen heraus radial nach innen in die Schaftaufnahme hinein gedrückt. Auf diese Weise können an einem in der Schaftaufnahme aufgenommenen Werkzeugschaft Klemmkräfte erzeugt werden, durch die der Werkzeugschaft mit der rotierenden Spannhülse mitgedreht wird.

Aus DE 20 2006 006 551 U1, das den Oberbegriff des Anspruchs 1 formt, ist ein medizinisch kosmetisches Bearbeitungsgerät mit einer Werkzeugaufnahme bekannt, das innerhalb einer zweiteiligen Spannhülse eine Spannzange vorsieht. Diese weist eine Axialbohrung zur Aufnahme eines Werkzeugschaftes sowie drei zueinander elastisch verschwenkbare Flügel auf, die zwischen sich Lagerausnehmungen für jeweils einen von drei Spannstiften bilden. Bei rotatorischem Antrieb der Spannhülse verklemmen sich diese Spannstifte zwischen einer polygonal geformten Innenwand der Spannhülse und dem Werkzeugschaft, um diesen ebenfalls in Rotation zu versetzen.

Nachteilig an der bekannten Werkzeugaufnahme ist, dass über die Axialbohrung Schmutzpartikel, wie insbesondere Staub, eindringen und sich an der Innenwand der Spannhülse festsetzen können. Dies kann dazu führen, dass die Spannstifte trotz Rotationsbewegung der Spannhülse ihre Klemmstellung nicht einnehmen können, sondern infolge der Verschmutzung in einem Bereich mit aufgeweitetem Querschnitt eingerückt bleiben. Hierdurch kann es passieren, dass keine ausreichenden Klemmkräfte zwischen der Spannhülse und dem in der Spannzange aufgenommenen Werkzeugschaft erzeugt werden und das Rotationswerkzeug bei Kontakt mit einer zu bearbeitenden Oberfläche aufgrund des dabei erzeugten Reibungswiderstandes stehen bleibt.

Die Aufgabe der Erfindung ist es, bei einer gattungsgemäßen Werkzeugaufnahme die genannten Nachteile zu vermeiden und das Risiko einer verschmutzungsbedingten Fehlfunktion zu minimieren.

Diese Aufgabe wird durch eine Werkzeugaufnahme mit den Merkmalen des Anspruchs 1 gelöst. Dabei bildet der Klemmelementträger zwischen der Schaftauf-nahme und den Klemmelementen eine umlaufend geschlossene Trägerwand. Hierdurch wird verhindert, dass die über die Schaftaufnahme eingetragenen Schmutzpartikel bis zu den Klemmelementen transportiert werden und deren Verlagerung zwischen einer Klemmstellung und einer Freigabestellung behindern. Vielmehr können die Klemmelemente durch die geschlossene Trägerwand wirksam gegenüber Schmutzpartikel, die während des Betriebs in die Schaftaufnahme gelangt sind, abgeschirmt werden.

Vorteilhafterweise ist die geschlossene Trägerwand zwischen der Schaftaufnahme und den Ausnehmungen des Klemmelementträgers flexibel ausgebildet. Hierdurch kann bei Rotation der Spannhülse über die Klemmelemente trotz der Zwischenlage der Trägerwand eine ausreichende Verengung der Schaftaufnahme erzielt beziehungsweise eine ausreichende Beaufschlagung eines in der Schaftaufnahme angeordneten Werkzeugschaftes durch Klemmkräfte erzielt werden, um diesen gegenüber der Spannhülse stabil festzulegen. Durch die wenigstens bereichsweise flexible und häutchenartige Ausbildung der Trägerwand kann dabei sichergestellt werden, dass die Klemmelemente nach Einschalten des Rotationsantriebs der Spannhülse bereits bei einer relativ geringen Umdrehungszahl aus ihrer Freigabestellung in die Klemmstellung verlagert werden.

Dabei ist es günstig, wenn die Trägerwand eine zylinderförmige Innenseite aufweist, um ein einfaches Einführen eines entsprechend zylindrisch geformten Werkzeugschaftes zu ermöglichen. Zudem kann hierdurch über die Länge der Schaftaufnahme ein vollständiger Flächenkontakt zwischen deren Innenseite mit dem Werkzeugschaft hergestellt werden, was eine besonders gute Abdichtung gegenüber Stäuben im Arbeitsbereich des Bearbeitungsgerätes gewährleistet.

In einer besonders vorteilhaften Ausführungsform bildet der Klemmelementträger an einer von einer Aufnahmeöffnung der Schaftaufnahme abgewandten Seite einen geschlossenen Aufnahmeboden. Auf diese Weise ist die Schaftaufnahme gegenüber dem Inneren der Werkzeugaufnahme vollständig verschlossen, so dass sich eindringende Schmutzpartikel in der Lageraufnahme ansammeln und beispielsweise mittel eines an die Form der Schaftaufnahme angepassten Spezialwerkzeuges von Zeit zu Zeit aus dieser entfernt werden können.

Zudem ist es günstig, wenn der Klemmelementträger aus Kunststoff gebildet ist, wodurch die Ausnehmungen und die geschlossene Trägerwand durch ein relativ einfach herzustellendes, einstückiges Bauteil gebildet werden können.

Vorteilhafterweise ist der Kunststoff durch ein Flourelastomer gebildet. Hierdurch kann der Klemmelementträger auch problemlos für Bearbeitungsgeräte mit einer Drehzahl von über 5000 U/min verwendet werden, da die Temperaturen, die bei diesen Drehzahlen entstehen können, problemlos aufgenommen werden können.

Ferner ist es von Vorteil, wenn der Klemmelementträger einen elastischen Klemmkragen bildet, der eine freie Klemmaufnahme umschließt, die in einem unbelasteten Zustand eine geringere Erstreckung ihres freien Querschnittes aufweist als ein Durchmesser des Werkzeugschaftes, der an der Werkzeugaufnahme aufgenommen werden soll. Hierdurch können durch die elastischen Rückstellkräfte der Klemmaufnahme zusätzliche Klemmkräfte auf den Werkzeugschaft aufgebracht werden, die das Rotationswerkzeug gegen axiales Verrutschen sichern und zusätzliche Haltekräfte zwischen der Spannhülse und dem Werkzeugschaft in Rotationsrichtung erzeugen.

Zudem ist es günstig, wenn an einem ersten Ende des Klemmelementträgers eine stirnseitig umlaufende Dichtungslippe vorgesehen ist. Auf diese Weise kann verhindert werden, dass Schmutzpartikel über die Innenseite der Spannhülse bis zu den Ausnehmungen für die Klemmelemente gelangen.

In den Figuren ist eine beispielhafte Ausführungsform der Erfindung dargestellt. Es zeigen:
- Figur 1: eine Ansicht einer erfindungsgemäßen Werkzeugaufnahme eines medizinischen oder kosmetischen Bearbeitungsgerätes,
- Figur 2: eine explodierte Ansicht der Werkzeugaufnahme nach Figur 1,
- Figur 3: eine vereinzelte Ansicht einer Spannzange der Werkzeugaufnahme nach Fig. 1,
- Figur 4a: eine geschnittene Ansicht durch eine Schnellspannvorrichtung der Werkzeugaufnahme in Ebene IV-IV aus Fig. 1 in einer Freigabestellung und
- Figur 4b: eine geschnittene Ansicht der Schnellspannvorrichtung aus Fig. 4a in einer Klemmstellung.

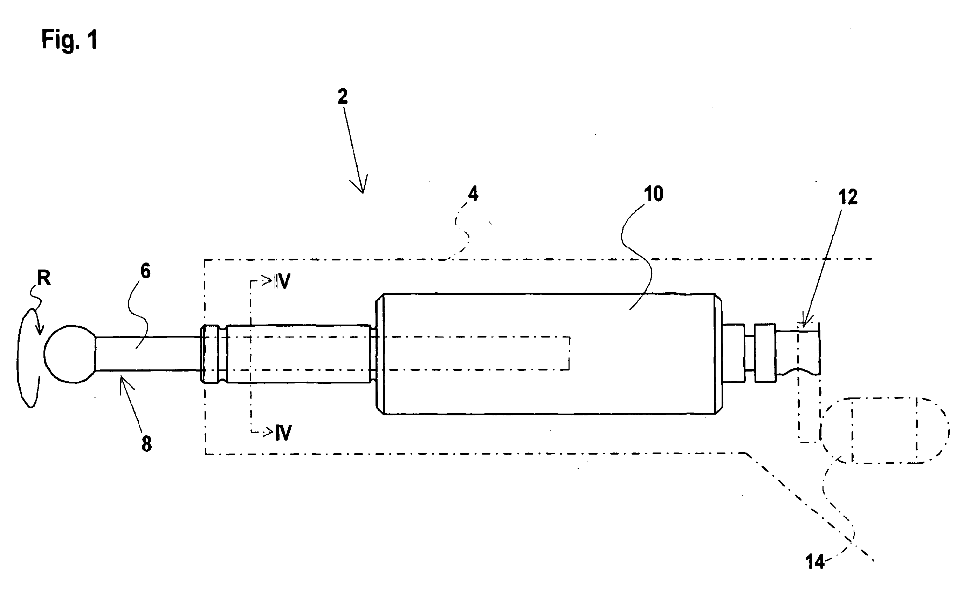

Fig. 1 zeigt eine Werkzeugaufnahme 2 eines angedeuteten Bearbeitungsgerätes 4 in Form eines Handstückes einer medizinischen oder kosmetischen Hautschleifmaschine, insbesondere für die Verwendung in der Pediküre oder in der Podologie. Die Werkzeugaufnahme 2 dient zur rotationsfesten Festlegung eines zylindrischen Werkzeugschaftes 6 eines Rotationswerkzeuges 8 in Form eines Hautschleiferbits.

Das Rotationswerkzeug 8 kann dabei von einem jeweiligen Benutzer werkzeugfrei durch bloßes Einstecken in eine Schnellspannvorrichtung 10 der Werkzeugaufnahme 2 angebracht werden. Die Schnellspannvorrichtung 10 ist wiederum über eine Drehmomentkopplung 12 mit einem Antriebsmotor 14 verbunden und kann somit von diesem in eine Rotationsrichtung R bewegt werden.

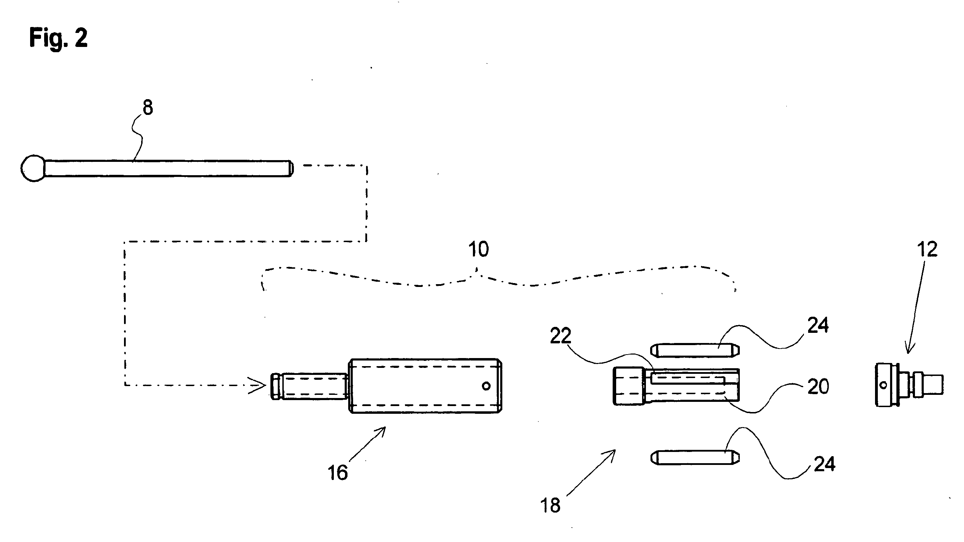

Wie aus Fig. 2 zu entnehmen ist, weist die Schnellspannvorrichtung 10 eine Spannhülse 16 auf, in der eine Spannzange 18 untergebracht ist. Diese Spannzange 18 weist einen Klemmelementträger 20 mit beispielhaft drei nutförmigen Ausnehmungen 22 auf, die jeweils zur Aufnahme eines walzenförmigen Klemm-elementes 24 dienen.

Der Klemmelementträger 20 ist hierbei bevorzugterweise einstückig aus einem Kunststoff, wie insbesondere ein Flourelastomer gebildet, was einerseits eine einfache Herstellung und Montage des Klemmelementträgers ermöglicht und andererseits eine hohe Temperaturbeständigkeit gewährleistet.

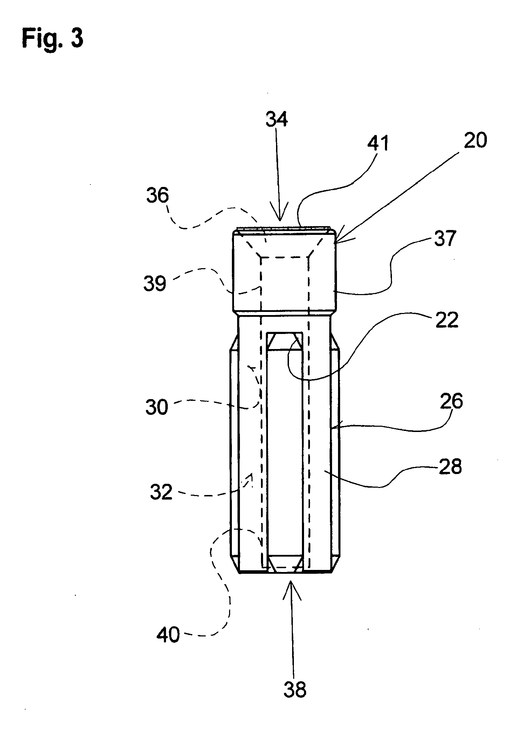

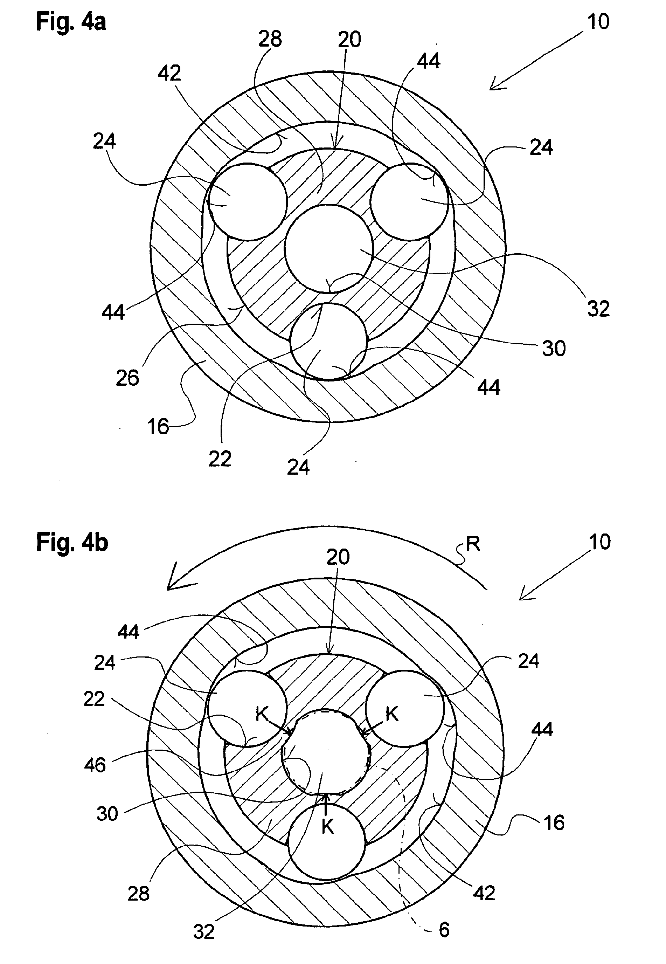

Wie insbesondere aus den Fig. 3 und 4 zu entnehmen ist, sind die nutförmigen Ausnehmungen 22 in eine Außenseite 26 einer Trägerwand 28 des Klemmelementträgers 20 eingelassen. Mit einer von der Außenseite 26 abgewandten Innenseite 30 der Trägerwand 28 begrenzt der Klemmelementträger 20 zudem eine mittige Schaftaufnahme 32, in der ein hinterer Teil des Werkzeugschaftes 6 aufgenommen werden kann.

Hierzu weist der Klemmelementträger 20, wie insbesondere aus Fig. 3 zu entnehmen ist, an einem ersten Ende 34 der Schaftaufnahme 32 eine Aufnahmeöffnung 36 auf. Diese ist beispielsweise an einem Klemmkragen 37 vorgesehen, der eine Klemmaufnahme 39 bildet, die in einem unbelasteten Zustand einen geringeren durchmesser aufweist als ein Durchmesser des für die Festlegung vorgesehenen Werkzeugschaftes 6 des Rotationswerkzeuges 8 (nicht dargestellt). Hierdurch wird der Werkzeugschaft 6 beim Einführen in die Aufnahmeöffnung 36 durch den Klemmkragen 37 sowohl in axialer Richtung als auch in Rotationsrichtung R festgeklemmt.

An einem vom ersten Ende 34 abgewandten zweiten Ende 38 bildet der Klemmelementträger 20 einen Aufnahmeboden 40 aus, so dass die Schaftaufnahme 32 bis auf die Aufnahmeöffnung 36 vollständig durch den Klemmelementträger 20 umschlossen ist.

Zudem ist am ersten Ende 34 des Klemmelementträgers 20 eine stirnseitig angeordnete umlaufende Dichtungslippe 41 vorgesehen. Hierdurch wird verhindert, dass im eingesetzten Zustand des Klemmelementträgers 20 in der Spannhülse 16 Schmutzpartikel, die in die Spannhülse 16 eindringen, am ersten Ende 34 zwischen Spannhülse 16 und Klemmelementträger 20 hindurch und an der Außenseite des Klemmelementträgers 20 entlang bis zu den Ausnehmungen 22 gelangen, wo sie die Verlagerung der Klemmelemente 24 behindern könnten.

Wie aus Fig. 4a und b zu entnehmen ist, liegen die am Klemmelementträger 20 gehaltenen Klemmelemente 24 an einer Innenwandung 42 der Spannhülse 16 an. In einer in Fig. 4a dargestellten Freigabestellung sind die Klemmelemente 24 in Einbuchtungen 44 der Innenwandung 42 eingerückt, die beispielsweise durch eine polygonale Profilierung der Innenwandung 42 erzeugt sein können. In dieser Freigabestellung weist die Innenseite 30 des aus Kunststoff gebildeten Klemmelementträgers 20, die die Schaftaufnahme 32 begrenzt, eine ebene Zylinderform auf.

Nach Einschalten des Antriebsmotors 14 wird die Spannhülse 16 dagegen in Rotationsrichtung R in Bewegung gesetzt und die Klemmelemente 24 werden sie auf Grund ihrer Trägheit aus den Einbuchtungen 44 heraus bewegt. Dabei werden sie von der Innenwandung 42 gegen einen jeweiligen Verformungsabschnitt 46 der Trägerwand 28 gedrückt, der sich zwischen der jeweiligen Ausnehmung 22 und der Innenseite 30 erstreckt. Die Verformungsabschnitte 46 weisen dabei eine derart geringe Stärke auf, dass sie bei Berücksichtigung der Materialeigenschaften des verwendeten Kunststoffes und der für den Betrieb des Bearbeitungsgerätes 4 vorgesehenen Umdrehungszahlen, durch den von den Klemmelementen 24 ausgeübten Druck verformt werden können. Hierdurch kann die Schaftaufnahme 32 mittels der Klemmelemente 24 unter Zwischenlage der häutchenartigen Verformungsabschnitte 46 der Trägerwand 28 verengt beziehungsweise ein in der Schaftaufnahme 32 gehaltener Werkzeugschaft 6 mit Klemmkräften K beaufschlagt werden, wie in Fig. 4b dargestellt.

Auf diese Weise wird der Werkzeugschaft 6 von der in Rotationsrichtung R rotierenden Spannhülse 16 ebenfalls in Rotationsrichtung R angetrieben.

## Patentansprüche

1. Werkzeugaufnahme (2) eines medizinischen oder kosmetischen Bearbeitungsgerätes (4)
mit einer Schnellspannvorrichtung (10) für die Festlegung eines Werkzeugschaftes (6) eines Rotationswerkzeuges (8),
die eine Schaftaufnahme (32) aufweist, deren freier Querschnitt für die Festlegung des eingeführten Werkzeugschaftes (6) durch Klemmelemente (24) verringerbar ist, die in Ausnehmungen (22) eines Klemmelementträgers (20) gehalten sind,
wobei der Klemmelementträger (20) innerhalb einer Spannhülse (16) angeordnet ist, die rotatorisch antreibbar ist und die auf Höhe der Klemmelemente (24) eine Innenwandung (42) mit Einbuchtungen (44) aufweist, in die die Klemmelemente (24) einrückbar sind,
**dadurch gekennzeichnet, dass** der Klemmelementträger (20) zwischen der Schaftaufnahme (32) und den Klemmelementen (24) eine umlaufend geschlossene Trägerwand (28) bildet.

2. Werkzeugaufnahme nach Anspruch 1, **dadurch gekennzeichnet, dass** die geschlossene Trägerwand (28) zwischen der Schaftaufnahme (32) und den Ausnehmungen (22) des Klemmelementträgers (20) flexibel ausgebildet ist.

3. Werkzeugaufnahme nach Anspruch 1 oder 2, **dadurch gekennzeichnet, dass** die Trägerwand (28) eine zylinderförmige Innenseite (30) aufweist.

4. Werkzeugaufnahme nach einem der Ansprüche 1 bis 3, **dadurch gekennzeichnet, dass** der Klemmelementträger (20) an einer von einer Aufnahmeöffnung (36) der Schaftaufnahme (32) abgewandten Seite einen geschlossenen Aufnahmeboden (40) bildet.

5. Werkzeugaufnahme nach einem der Ansprüche 1 bis 4, **dadurch gekennzeichnet, dass** der Klemmelementträger (20) aus Kunststoff gebildet ist.

6. Werkzeugaufnahme nach Anspruch 5, **dadurch gekennzeichnet, dass** der Kunststoff durch Flourelastomer gebildet ist.

7. Werkzeugaufnahme nach Anspruch 5 oder 6, **dadurch gekennzeichnet, dass** der Klemmelementträger (20) einen Klemmkragen (37) bildet, der eine Klemmaufnahme (39) umschließt, die in einem unbelasteten Zustand eine geringere Erstreckung aufweist als ein Durchmesser des für die Festlegung vorgesehenen Werkzeugschaftes (6).

8. Werkzeugaufnahme nach einem der Ansprüche 1 bis 7, **dadurch gekennzeichnet, dass** an einem ersten Ende (34) des Klemmelementträgers (20) eine stirnseitig umlaufende Dichtungslippe (41) vorgesehen ist.

## Claims

1. Tool receptacle (2) of a medical or cosmetic processing device (4)
comprising a quick clamping device (10) for fastening a tool shaft (6) of a rotary tool (8),
wherein on said quick clamping device a shaft receptacle (32) is provided, the free cross-section of which can be reduced by clamping elements (24) retained in holes (22) of a clamping-element carrier (20) in order to fasten the inserted tool shaft (6),
wherein the clamping-element carrier (20) is arranged inside a clamping sleeve (16), which is rotationally driven and which has an inner wall (42) at the height of the clamping elements (24), the inner wall having recesses (44), into which the clamping elements (24) can be moved,
**characterised in that** the clamping-element carrier (20) forms a carrier wall (28) closed all around between the shaft receptacle (32) and the clamping elements (24).

2. Tool receptacle according to claim 1, **characterised in that** the closed carrier wall (28) is flexibly designed between the shaft receptacle (32) and the holes (22) of the clamping-element carrier (20).

3. Tool receptacle according to claim 1 or 2, **characterised in that** the carrier wall (28) has a cylindrical inside (30).

4. Tool receptacle according to any one of claims 1 to 3, **characterised in that** the clamping-element carrier (20) forms a closed receptacle bottom (40) on a side that faces away from a receptacle opening (36) of the shaft receptacle (32).

5. Tool receptacle according to any one of claims 1 to 4, **characterised in that** the clamping-element carrier (20) is formed from plastic.

6. Tool receptacle according to claim 5, **characterised in that** the plastic is formed from fluoroelastomer.

7. Tool receptacle according to claim 5 or 6, **characterised in that** the clamping-element carrier (20) forms a clamping collar (37) which encloses a clamping receptacle (39) which, in an unloaded state, has a smaller extension than a diameter of the tool shaft (6) intended for fastening.

8. Tool receptacle according to any one of claims 1 to 7, **characterised in that** a circumferential front sealing lip (41) is provided at a first end (34) of the clamping-element carrier (20).

## Revendications

1. Porte-outil (2) d'un appareil (4) de traitement médical ou cosmétique
comportant un dispositif de serrage rapide (10) pour la fixation d'une tige d'outil (6) d'un outil rotatif (8),
ledit dispositif de serrage rapide (10) présente un logement de tige (32), dont la section transversale libre est réductible par l'intermédiaire d'éléments de blocage (24) pour la fixation de la tige d'outil insérée (6), lesdits éléments de blocage (24) sont maintenus dans des évidements (22) d'un support d'éléments de blocage (20),
où le support d'éléments de blocage (20) est disposé à l'intérieur d'un manchon de serrage (16), qui peut être entraîné en rotation et qui au niveau des éléments de blocage (24) présente une paroi intérieure (42) avec des creux dans lesquels les éléments de blocage (24) peuvent être repoussés,
**caractérisé en ce que** le support d'éléments de blocage (20) forme entre le logement de tige (32) et les éléments de blocage (24) une paroi de support périphérique fermée (28).

2. Porte-outil selon la revendication 1, **caractérisé en ce que** la paroi de support fermée (28) est flexible entre le logement de tige (32) et les évidements (22) du support d'éléments de blocage (20).

3. Porte-outil selon la revendication 1 ou 2, **caractérisé en ce que** la paroi de support (28) présente une face interne (30) cylindrique.

4. Porte-outil selon l'une des revendications 1 à 3, **caractérisé en ce que** le support d'éléments de blocage (20) forme un fond de logement (40) fermé en un côté opposé à une ouverture (36) du logement de tige (32).

5. Porte-outil selon l'une des revendications 1 à 4, **caractérisé en ce que** le support d'éléments de blocage (20) est réalisé en matière plastique.

6. Porte-outil selon la revendication 5, **caractérisé en ce que** la matière plastique est constituée d'élastomère fluoré.

7. Porte-outil selon la revendication 5 ou 6, **caractérisé en ce que** le support d'éléments de blocage (20) forme un collet de blocage (37), qui entoure un logement de blocage (39) présentant dans un état non chargé une étendue inférieure à un diamètre de tige d'outil (6) prévue pour la fixation.

8. Porte-outil selon l'une des revendications 1 à 7, **caractérisé en ce qu'**un joint d'étanchéité annulaire(41) est prévu en face avant à une première extrémité (34) du support d'éléments de blocage (20).
